# FASCICULE DE BREVET EUROPEEN

(11) **EP 1 244 792 B1**
(45) Date de publication et mention de la délivrance du brevet: **18.03.2009**
(21) Numéro de dépôt: 01903880.1
(22) Date de dépôt: 05.01.2001
(51) Int. Cl.: C12N 15/53, C12N 15/82, C12N 9/02, A01H 5/00

(54) **PROCEDE D'OBTENTION DE PLANTES A TENEUR ENRICHIE EN CYSTEINE ET GLUTATHION**
VERFAHREN ZUR HESTELLUNG VON PFLANZEN MIT ERHÖHTEM GEHALT AN CYSTEIN UND GLUTATHION
METHOD FOR OBTAINING PLANTS ENRICHED IN CYSTEINE AND GLUTATHIONE CONTENT

(30) Priorité: 06.01.2000 FR 0000139
(43) Date de publication de la demande: 02.10.2002
(73) Titulaire: Biogemma, 75001 Paris (FR)
(72) Inventeur: BRUNOLD, Christian, CH-3084 WABERN (CH); PEREZ, Pascual, F-63450 Chanonat (FR)
(74) Mandataire: Flesselles, Bruno F.G.
(86) Numéro de dépôt international: PCT/FR2001/000036
(87) Numéro de publication internationale: WO 2001/049855

(56) Documents cités:
- WO-A-00/04161
- WO-A-00/49165
- HELL R: "MOLECULAR PHYSIOLOGY OF PLANT SULFUR METABOLISM" PLANTA,SPRINGER VERLAG,DE, vol. 202, 1997, pages 138-148, XP000856032 ISSN: 0032-0935
- PRIOR A ET AL: "STRUCTURAL AND KINETIC PROPERTIES OF ADENYLYL SULFATE REDUCTASE FROM CATHARANTHUS ROSEUS CELL CULTURES" BIOCHIMICA ET BIOPHYSICA ACTA,AMSTERDAM,NL, vol. 1430, 1999, pages 25-38, XP000853820 ISSN: 0006-3002 & DATABASE EMBL [en ligne] ACCESSION NO: U63784, 11 août 1996 (1996-08-11)
- HEISS ET AL: "cloning sulfur assimilation genes from Brassica juncea L.: cadmium differentially affects the expression of a putative low-affinity sulfate transporter and isoforms of ATP sulfurylase and APS reductase" PLANT MOLECULAR BIOLOGY,NIJHOFF PUBLISHERS, DORDRECHT,NL, vol. 39, mars 1999 (1999-03), pages 847-857, XP002122448 ISSN: 0167-4412 & DATABASE EMBL [en ligne] ACCESSION NO: AJ001208, 16 décembre 1998 (1998-12-16) & DATABASE EMBL [en ligne] ACCESSION NO: AJ001207, 16 décembre 1998 (1998-12-16)
- SEYTA ET AL: "sulfate reduction in higher plants: molecular evidence for a novel 5'-adenylylsulfate reductase" PROCEEDINGS OF THE NATIONAL ACADEMY OF SCIENCES OF USA,NATIONAL ACADEMY OF SCIENCE. WASHINGTON,US, vol. 93, novembre 1996 (1996-11), pages 13383-13388, XP002122451 ISSN: 0027-8424 & DATABASE EMBL [en ligne] ACCESSION NO: U56922, 31 mai 1996 (1996-05-31) & DATABASE EMBL [en ligne] ACCESSION NO: U56921, 31 mai 1996 (1996-05-31)
- GUTIERREZ-MARCOS ET AL: "three members of a novel small gene-family from Arabidopsis able to complement functionally an Escherichia coli mutant defective in PAPS reductase activity encode proteins with thioredoxin-like domain and APS reductase activity" PROCEEDINGS OF THE NATIONAL ACADEMY OF SCIENCES OF USA,NATIONAL ACADEMY OF SCIENCE. WASHINGTON,US, vol. 93, novembre 1996 (1996-11), pages 13377-13382, XP002122426 ISSN: 0027-8424 & DATABASE EMBL [en ligne] ACCESSION NO: U53864, 14 décembre 1996 (1996-12-14) & DATABASE EMBL [en ligne] ACCESSION NO: U53866, 14 décembre 1996 (1996-12-14) & DATABASE EMBL [en ligne] ACCESSION NO: U53865, 14 décembre 1996 (1996-12-14)
- SUTER ET AL: "ADENOSINE 5'-PHOSPHOSULFATE SULFOTRANSFERASE AND ADENOSINE 5'-PHOSPHOSULFATE REDUCTASE ARE IDENTICAL ENZYMES" JOURNAL OF BIOLOGICAL CHEMISTRY,AMERICAN SOCIETY OF BIOLOGICAL CHEMISTS, BALTIMORE, MD,US, vol. 275, no. 2, 14 janvier 2000 (2000-01-14), pages 930-936, XP002142803 ISSN: 0021-9258 & DATABASE EMBL [en ligne] ACCESSION NO: AJ249831, 14 janvier 2000 (2000-01-14)
- BICK ET AL: "Glutaredoxin function for the carboxyl-terminal domain of the plant-type 5'-adenylylsulfate reductase" PROCEEDINGS OF THE NATIONAL ACADEMY OF SCIENCES OF USA,NATIONAL ACADEMY OF SCIENCE. WASHINGTON,US, vol. 95, juillet 1998 (1998-07), pages 8404-8409, XP002122450 ISSN: 0027-8424
- NEUENSCHWANDER U ET AL: "REGULATION OF SULFATE ASSIMILATION BY LIGHT AND O ACETYL-L-SERINE IN LEMNA-MINOR L" PLANT PHYSIOLOGY (BETHESDA), vol. 97, no. 1, 1991, pages 253-258, XP002148495 ISSN: 0032-0889
- SAITO K: "Molecular aspects of sulfur assimilation and acclimation to sulfur supply in plants" STRESS RESPONSES OF PHOTOSYNTHETIC ORGANISMS,NL,ELSEVIER SCIENCE, AMSTERDAM, 1998, pages 215-226, XP002121796
- SAITO ET AL: "modulation of cysteine biosynthesis in chloroplasts of transgenic tobacco overexpressing cysteine synthase (O-Acetylserine(thiol)-lyase)" PLANT PHYSIOLOGY,US,AMERICAN SOCIETY OF PLANT PHYSIOLOGISTS, ROCKVILLE, MD, no. 106, 1994, pages 887-895, XP002078205 ISSN: 0032-0889
- BRUNOLD C ET AL: "III. REGULATION OF SULFUR METABOLISM IN PLANTS: FIRST MOLECULAR APPROACHES" PROGRESS IN BOTANY - FORTSCHRITTE DER BOTANIK,SPRINGER, BERLIN,DE, vol. 58, 1997, pages 164-186, XP000856058 ISSN: 0340-4773
- BICK J A ET AL: "PLANT SULFUR METABOLISM - THE REDUCTION OF SULFATE TO SULFITE" CURRENT OPINION IN PLANT BIOLOGY,GB,QUADRANT SUBSCRIPTION SERVICES, vol. 1, no. 3, juin 1998 (1998-06), pages 240-244, XP000853523 ISSN: 1369-5266
- DATABASE WPI Section Ch, Week 199412 Derwent Publications Ltd., London, GB; Class C06, AN 1994-094834 XP002148496 & JP 06 038770 A (MITSUBISHI CORP), 15 février 1994 (1994-02-15)

### LISTE DE SEQUENCES

<110> BIOGEMMA
<120> Procédé d'obtention de plantes à teneur enrichie en cystéine et glutathion
<130> BFF 99/0652
<140>
   <141>
<160> 11
<170> PatentIn Ver. 2.1
<210> 1
   <211> 1380
   <212> ADN
   <213> Lemna minor
<220>
   <221> CDS
   <222> (1)..(1377)
<400> 1
   atg tcg gcc gca gca gca act atg gcg ggc tcg ctc tct tca cac acg    48
   Met Ser Ala Ala Ala Ala Thr Met Ala Gly Ser Leu Ser Ser His Thr
     1               5                  10                  15
   ctc tcc tcc cgc gaa ttt tca gga gga ttg cag gtc agc tct acg agg    96
   Leu Ser Ser Arg Glu Phe Ser Gly Gly Leu Gln Val Ser Ser Thr Arg
                20                  25                  30
   gtg agg aaa ttg cag agg gga gcg ttt gct ccg atg cgg cct ctg tgt   144
   Val Arg Lys Leu Gln Arg Gly Ala Phe Ala Pro Met Arg Pro Leu Cys
            35                  40                  45
   gcg gtg gat cca ggg agg aag agc cag tcg cca gtg gcg ccg ctg gcc   192
   Ala Val Asp Pro Gly Arg Lys Ser Gln Ser Pro Val Ala Pro Leu Ala
        50                  55                  60
   gcc gtg ccg tcg tcc acc tct gat gcc gtg act ctt gtc caa gca gca   240
   Ala Val Pro Ser Ser Thr Ser Asp Ala Val Thr Leu Val Gln Ala Ala
    65                  70                  75                  80
   gaa acg agc acc gtt gac gtg gtt gat ttt gag aag ctc gcg agc gag   288
   Glu Thr Ser Thr Val Asp Val Val Asp Phe Glu Lys Leu Ala Ser Glu
                    85                  90                  95
   cta gag cgc gca tcg cct ctc gag att atg gac aag gct ctg gag atg   336
   Leu Glu Arg Ala Ser Pro Leu Glu Ile Met Asp Lys Ala Leu Glu Met
               100                 105                 110
   ttc ggt aac gat att gcc atc gcc ttc agc ggg gcg gag gac gtt gct   384
   Phe Gly Asn Asp Ile Ala Ile Ala Phe Ser Gly Ala Glu Asp Val Ala
           115                 120                 125
   ctg atc gag tac gca cgc cta acc gga cga cct ttc agg gtg ttc agt   432
   Leu Ile Glu Tyr Ala Arg Leu Thr Gly Arg Pro Phe Arg Val Phe Ser
       130                 135                 140
   ctc gac acc ggc agg cta aac cca gag aca tac cga ttc ttc gat gaa     480
   Leu Asp Thr Gly Arg Leu Asn Pro Glu Thr Tyr Arg Phe Phe Asp Glu
   145                 150                 155                 160
   gta gag aag cgc tac aac atc cgc atc gag tac atg ttc ccc gac gcg     528
   Val Glu Lys Arg Tyr Asn Ile Arg Ile Glu Tyr Met Phe Pro Asp Ala
                   165                 170                 175
   gtg gaa gtc caa gcc ctc gtg cga agc aaa ggc ctc ttt tcc ttc tac     576
   Val Glu Val Gln Ala Leu Val Arg Ser Lys Gly Leu Phe Ser Phe Tyr
               180                 185                 190
   gag gac ggg cac cag gag tgc tgc aga gtg aga aag gtg agg ccc ctt     624
   Glu Asp Gly His Gln Glu Cys Cys Arg Val Arg Lys Val Arg Pro Leu
           195                 200                 205
   aga aga gct ctc aga ggc ctc aga gcc tgg atc acc ggc cag cgc aag     672
   Arg Arg Ala Leu Arg Gly Leu Arg Ala Trp Ile Thr Gly Gln Arg Lys
       210                 215                 220
   gac cag tct ccc ggc acc cga gcc agc gtt ccc acc gtc cag gtg gat     720
   Asp Gln Ser Pro Gly Thr Arg Ala Ser Val Pro Thr Val Gln Val Asp
   225                 230                 235                 240
   cct tcg ttc gag gga ttt gaa ggc gga aca ggc agc tta atc aaa tgg     768
   Pro Ser Phe Glu Gly Phe Glu Gly Gly Thr Gly Ser Leu Ile Lys Trp
                   245                 250                 255
   aac ccc gtg gcg aac gta gac ggg cag gat atc tgg aga ttc ctg aga     816
   Asn Pro Val Ala Asn Val Asp Gly Gln Asp Ile Trp Arg Phe Leu Arg
               260                 265                 270
   acg atg gcc gtt cca gtg aac tcc ctc cac tcc caa ggg tac gtg tca     864
   Thr Met Ala Val Pro Val Asn Ser Leu His Ser Gln Gly Tyr Val Ser
           275                 280                 285
   atc ggc tgc gag cca tgc acg cgg ccg gtc cta cca gga cag cac gag     912
   Ile Gly Cys Glu Pro Cys Thr Arg Pro Val Leu Pro Gly Gln His Glu
       290                 295                 300
   agg gaa gga agg tgg tgg tgg gag gac gcg aaa gcc aag gaa tgc ggg     960
   Arg Glu Gly Arg Trp Trp Trp Glu Asp Ala Lys Ala Lys Glu Cys Gly
   305                 310                 315                 320
   ctc cac aag ggc aac atc aag caa gac gag tcc gtg cct ccc tcc agc     1008
   Leu His Lys Gly Asn Ile Lys Gln Asp Glu Ser Val Pro Pro Ser Ser
                   325                 330                 335
   aac ggc aac ggc acc gcg ccg tcg gat ctc ttc tcc gtc gtt tct ctg     1056
   Asn Gly Asn Gly Thr Ala Pro Ser Asp Leu Phe Ser Val Val Ser Leu
               340                 345                 350
   agc cgg cca ggc atg gag aac ttg ctc cgc ctg gag agc cgg aag gac     1104
   Ser Arg Pro Gly Met Glu Asn Leu Leu Arg Leu Glu Ser Arg Lys Asp
           355                 360                 365
   tcg tgg ctt gtc gtg ctc tat gct ccc tgg tgc cgc ttc tgc cag ggg    1152
   Ser Trp Leu Val Val Leu Tyr Ala Pro Trp Cys Arg Phe Cys Gln Gly
       370                 375                 380
   atg gag gag tcg ttc aac gag gtg gca gga gta ctc ggc ggc gac tca    1200
   Met Glu Glu Ser Phe Asn Glu Val Ala Gly Val Leu Gly Gly Asp Ser
   385                 390                 395                 400
   gga gtg aag gtg ggc aaa ttc aga gcc gac gga gac gcg aga gct ttc    1248
   Gly Val Lys Val Gly Lys Phe Arg Ala Asp Gly Asp Ala Arg Ala Phe
                   405                 410                 415
   gcg gag gag atc ggg ctg gtg agt ttt ccc acc ata ctc ttc ttc ccg    1296
   Ala Glu Glu Ile Gly Leu Val Ser Phe Pro Thr Ile Leu Phe Phe Pro
               420                 425                 430
   aag cac gcg tcg aaa ccg gtc aag tac ccg tcg gag cag aga gac gtc    1344
   Lys His Ala Ser Lys Pro Val Lys Tyr Pro Ser Glu Gln Arg Asp Val
           435                 440                 445
   gac tct ctg ctc gcc ttc gtc aac gcc ctt cgc tga                    1380
   Asp Ser Leu Leu Ala Phe Val Asn Ala Leu Arg
       450                 455
<210> 2
   <211> 459
   <212> PRT
   <213> Lemna minor
<400> 2
   Met Ser Ala Ala Ala Ala Thr Met Ala Gly Ser Leu Ser Ser His Thr
    1               5                  10                  15
   Leu Ser Ser Arg Glu Phe Ser Gly Gly Leu Gln Val Ser Ser Thr Arg
               20                  25                  30
   Val Arg Lys Leu Gln Arg Gly Ala Phe Ala Pro Met Arg Pro Leu Cys
           35                  40                  45
   Ala Val Asp Pro Gly Arg Lys Ser Gln Ser Pro Val Ala Pro Leu Ala
       50                  55                  60
   Ala Val Pro Ser Ser Thr Ser Asp Ala Val Thr Leu Val Gln Ala Ala
   65                  70                  75                  80
   Glu Thr Ser Thr Val Asp Val Val Asp Phe Glu Lys Leu Ala Ser Glu
                   85                  90                  95
   Leu Glu Arg Ala Ser Pro Leu Glu Ile Met Asp Lys Ala Leu Glu Met
               100                 105                 110
   Phe Gly Asn Asp Ile Ala Ile Ala Phe Ser Gly Ala Glu Asp Val Ala
           115                 120                 125
   Leu Ile Glu Tyr Ala Arg Leu Thr Gly Arg Pro Phe Arg Val Phe Ser
       130                 135                 140
   Leu Asp Thr Gly Arg Leu Asn Pro Glu Thr Tyr Arg Phe Phe Asp Glu
   145                 150                 155                 160
   Val Glu Lys Arg Tyr Asn Ile Arg Ile Glu Tyr Met Phe Pro Asp Ala
                   165                 170                 175
   Val Glu Val Gln Ala Leu Val Arg Ser Lys Gly Leu Phe Ser Phe Tyr
               180                 185                 190
   Glu Asp Gly His Gln Glu Cys Cys Arg Val Arg Lys Val Arg Pro Leu
           195                 200                 205
   Arg Arg Ala Leu Arg Gly Leu Arg Ala Trp Ile Thr Gly Gln Arg Lys
       210                 215                 220
   Asp Gln Ser Pro Gly Thr Arg Ala Ser Val Pro Thr Val Gln Val Asp
   225                 230                 235                 240
   Pro Ser Phe Glu Gly Phe Glu Gly Gly Thr Gly Ser Leu Ile Lys Trp
                   245                 250                 255
   Asn Pro Val Ala Asn Val Asp Gly Gln Asp Ile Trp Arg Phe Leu Arg
               260                 265                 270
   Thr Met Ala Val Pro Val Asn Ser Leu His Ser Gln Gly Tyr Val Ser
           275                 280                 285
   Ile Gly Cys Glu Pro Cys Thr Arg Pro Val Leu Pro Gly Gln His Glu
       290                 295                 300
   Arg Glu Gly Arg Trp Trp Trp Glu Asp Ala Lys Ala Lys Glu Cys Gly
   305                 310                 315                 320
   Leu His Lys Gly Asn Ile Lys Gln Asp Glu Ser Val Pro Pro Ser Ser
                   325                 330                 335
   Asn Gly Asn Gly Thr Ala Pro Ser Asp Leu Phe Ser Val Val Ser Leu
               340                 345                 350
   Ser Arg Pro Gly Met Glu Asn Leu Leu Arg Leu Glu Ser Arg Lys Asp
           355                 360                 365
   Ser Trp Leu Val Val Leu Tyr Ala Pro Trp Cys Arg Phe Cys Gln Gly
       370                 375                 380
   Met Glu Glu Ser Phe Asn Glu Val Ala Gly Val Leu Gly Gly Asp Ser
   385                 390                 395                 400
   Gly Val Lys Val Gly Lys Phe Arg Ala Asp Gly Asp Ala Arg Ala Phe
                   405                 410                 415
   Ala Glu Glu Ile Gly Leu Val Ser Phe Pro Thr Ile Leu Phe Phe Pro
               420                 425                 430
   Lys His Ala Ser Lys Pro Val Lys Tyr Pro Ser Glu Gln Arg Asp Val
           435                 440                 445
   Asp Ser Leu Leu Ala Phe Val Asn Ala Leu Arg
       450                 455
<210> 3
   <211> 20
   <212> ADN
   <213> Lemna minor
<400> 3
   actggtcctt gcgctggccg 20
<210> 4
   <211> 38
   <212> ADN
   <213> Lemna minor
<400> 4
   gaccacgcgt atcgatgtcg actttttttt tttttttt 38
<210> 5
   <211> 22
   <212> ADN
   <213> Lemna minor
<400> 5
   gtcgacatcg atacgcgtgg tc 22
<210> 6
   <211> 20
   <212> ADN
   <213> Lemna minor
<400> 6
   gtgatccagg ctctgaggcc 20
<210> 7
   <211> 22
   <212> ADN
   <213> Lemna minor
<400> 7
   tctgagagct cttctaaggg gc 22
<210> 8
   <211> 20
   <212> ADN
   <213> Lemna minor
<400> 8
   accaggacag cacgagaggg 20
<210> 9
   <211> 20
   <212> ADN
   <213> Lemna minor
<400> 9
   aaggtggtgg tgggaggacg 20
<210> 10
   <211> 39
   <212> ADN
   <213> Lemna minor
<400> 10
   cgtctagagg ccatggcggc cgcagcagca actatggcg 39
<210> 11
   <211> 34
   <212> ADN
   <213> Lemna minor
<400> 11
   aaagaattcc actctagaag cgaagagaag aggc 34

## Description

La présente invention a trait à un procédé d'obtention de plantes à teneur enrichie en cystéine et glutathion, par transgénèse.

Les plantes supérieures utilisent le sulfate inorganique pour satisfaire leurs besoins nutritionnels en soufre. Le soufre est assimilé par les plantes par réduction du sulfate en cystéine (Bick et Leustek, 1998). Cet acide aminé intervient alors dans de nombreuses fonctions dans la plante (Rennenberg et al, 1990 ; Schmidt et al, 1992), et participe notamment dans la synthèse des protéines soufrées telles que les protéines membranaires.

Le soufre réduit se retrouve également dans la méthionine et plusieurs métabolites, tels que le glutathion, dont la voie de biosynthèse est liée à celle de la cystéine (Brunold et al, 1997).

Le glutathion, qui est un tripeptide (γ-glutamylcystéineylglycine), joue un rôle dans la régulation de la nutrition soufrée mais aussi dans la défense des plantes contre divers stress, comme la sécheresse, le froid, la chaleur et la détoxification des xénobiontes comme des métaux lourds (Rennenberg et al, 1994 ; May et al, 1998).

Les voies d'assimilation du sulfate dans les plantes sont relativement complexes et non encore totalement élucidées. Elles font intervenir un certain nombre d'enzymes, dont l'Adénosine 5'-Phosphosulfate Réductase (APR).

Prior A et al. (Biochimica et Biophysica Acta 1430 (1999) 25-38) décrit l'isolement d'une APS reductase à partir de *Catharanthus roseus*, ainsi que la démonstration in vitro de son activité enzymatique.

Setya et al. (PNAS Vol. 93, pp 13383-13388, November 1996) décrit le clonage d'APS sulfotransferase d'*Arabidopsis thaliana* (APR-1, -2, et -3) ainsi que la complémentation de mutants d'*E.coli* déficients en cystéine.

Gutierrez et al. (PNAS Vol. 93, pp 13377-13382, November 1996) décrit trois ADNc d'*Arabidopsis thaliana* ayant une activité APS reductase (Prh-19, -26 et -43) capables de complémenter fonctionnellement un mutant cysH d'*E. coli* déficient en PAPS reductase.

Les auteurs de la présente invention ont maintenant mis au point un procédé d'obtention de plantes à teneur enrichie en cystéine et/ou glutathion, par transgénèse et surexpression d'un gène codant pour une enzyme APR de *Lemna minor*, de SEQ ID N°1.

L'intérêt d'utiliser cette enzyme de *Lemna* est multiple : elle présente notamment une forte activité enzymatique (quantifiée à 40µmoles par minute par mg de protéine) et est stable (jusqu'à 13 jours à 0-4°C).

L'invention a plus particulièrement pour objet un procédé d'obtention d'une plante présentant une teneur enrichie en cystéine et/ou glutathion, comprenant les étapes consistant à :
- transformer au moins une cellule de plante avec un vecteur contenant une cassette d'expression comprenant une séquence codant pour une Adénosine 5'-Phosphosulfate Réductase (APR) de *Lemna minor* de SEQ ID N°1;
- cultiver la cellule ainsi transformée de manière à générer une plante contenant dans son génome ladite cassette d'expression.

Ladite séquence codant pour l'APR est une séquence de la plante aquatique *Lemna minor* (lentille d'eau), telle que la séquence SEQ ID n° 1 en annexe.

L'acide nucléique codant pour l'APR selon l'invention est inséré dans une construction nucléotidique, appelée cassette d'expression, dans laquelle ladite séquence codante est liée de manière opérante à des éléments permettant son expression et éventuellement sa régulation.

Parmi ces éléments, on peut citer notamment les promoteurs, les activateurs et les terminateurs de transcription.

De manière générale, on peut utiliser par exemple un promoteur fort, tel que le promoteur 35S (Kay et al, 1987) ou le promoteur actine du riz suivi de l'intron actin de riz (PAR-IAR) contenu dans le plasmide pAct1-F4 (Mc Elroy et al, 1991), ou un promoteur tissu-spécifique. On peut en particulier utiliser un promoteur permettant l'expression de la séquence de l'APR dans les semences de la plante obtenue. A titre d'exemple, on peut citer le promoteur HMGW de blé, qui permet une expression dans les graines.

Parmi les terminateurs utilisables dans les constructions de l'invention, on peut citer notamment l'extrémité 3' du gène de la nopaline synthase d'*Agrobacterium tumefaciens*.

La cassette d'expression est insérée dans un vecteur nucléotidique, tel qu'un plasmide, qui peut en outre comprendre un gène marqueur, par exemple un gène permettant de sélectionner une plante transformée d'une plante qui ne contient pas l'ADN étranger transfecté. Comme gène marqueur, on peut citer notamment un gène conférant une résistance à un antibiotique (Herrera-Estrella et al, EMBO J. 2, 987-995 (1983) ou une résistance à un herbicide (EP 242 246).

Le vecteur ainsi construit est utilisable pour la transformation de cellules hôtes, selon les techniques connues de l'homme du métier.

On peut citer notamment les méthodes de transfert direct de gènes aux cellules végétales telles que la microinjection directe dans des embryoïdes de plante (Neuhaus et Coll., 1987), l'infiltration sous vide (Bechtold et al., 1993) ou l'électroporation (Chupeau et Coll., 1989) ou encore la précipitation directe au moyen de PEG (Schocher et Coll., 1986) ou le bombardement par canon de particules recouvertes de l'ADN plasmidique d'intérêt (Fromm M. et al., 1990).

Selon un autre mode de réalisation du procédé de l'invention, les cellules végétales sont transformées par un vecteur tel que défini précédemment, transféré dans un hôte cellulaire susceptible d'infecter lesdites cellules végétales en permettant l'intégration dans le génome de ces dernières, des séquences nucléotidiques d'intérêt initialement contenues dans le génome de vecteur susmentionné. Avantageusement, l'hôte cellulaire utilisé est une souche bactérienne, telle que *Agrobacterium tumefaciens*, notamment selon la méthode décrite dans l'article d'An et al, (1986), ou encore *Agrobacterium rhizogenes*, notamment selon la méthode décrite dans l'article de Guerche et al, (1987).

Par exemple, la transformation des cellules végétales peut être réalisée par le transfert de la région T du plasmide circulaire extra-chromosomique inducteur de tumeurs Ti d'*Agrobacterium tumefaciens*, en utilisant un système binaire (Watson et al, 1994). Pour ce faire, deux vecteurs sont construits. Dans l'un de ces vecteurs, la région T a été éliminée par délétion, à l'exception des bordures droite et gauche, un gène marqueur étant inséré entre eux pour permettre la sélection dans les cellules de plantes. L'autre partenaire du système binaire est un plasmide Ti auxiliaire, plasmide modifié qui n'a plus de région T mais contient toujours les gènes de virulence *vir*, nécessaires à la transformation de la cellule végétale.

Selon un mode préféré, on peut utiliser la méthode décrite par lshida et al (1996) pour la transformation des Monocotylédones.

Selon un autre protocole, la transformation est réalisée selon la méthode décrite par Finer et al, (1992) utilisant le canon à particules de tungstène ou d'or.

L'invention a également pour objet une plante ou partie de plante, notamment semence, grain, fruit, feuille ou tubercule, susceptible d'être obtenue par le procédé décrit plus haut.

Il peut s'agir de plantes de grandes cultures (maïs, blé, colza, tournesol, pois, soja, orge..) ou des plantes potagères et fleurs. Préférentiellement, on peut choisir des plantes connues pour contenir de grandes réserves protéiques, notamment les plantes céréalières ou les légumineuses telles que la pomme de terre.

Les plantes transgéniques hybrides obtenues par le croisement d'au moins une plante selon l'invention, font aussi partie de l'invention.

Conformément à la présente invention, un acide nucléique codant pour une Adénosine 5'-phosphosulfate Réductase (APR) est utilisée pour l'obtention d'une plante transgénique présentant une teneur enrichie en cystéine et/ou glutathion, par rapport à une plante non transformée.

Des plantes transgéniques produisant deux fois plus de cystéine et deux fois plus de glutathion que des plantes non transformées ont ainsi pu être obtenues au moyen du procédé de l'invention.

Les plantes avec une teneur enrichie en glutathion présentent notamment l'avantage d'être plus résistantes aux basses températures. La résistance au froid d'un génotype non résistant à une température de 12°C est augmentée de 30 % lorsque le glutathion est augmenté de 50 %.

L'accroissement de la teneur en cystéine d'une plante ou partie de plante rend celle-ci particulièrement intéressante pour la préparation de produits dérivés, notamment de produits alimentaires, de préférence pour l'alimentation animale. Les produits qui contiennent une plante ou partie de plante transgénique enrichie en cystéine et/ou glutathion, font d'ailleurs également partie de l'invention.

De manière avantageuse, les cellules végétales peuvent être cotransformées avec un acide nucléique codant pour une sérine Acétyltransférase (SAT), enzyme qui intervient dans le métabolisme de la cystéine (Murillo et al, 1995 ; Saito et al, 1995 et Roberts et al, 1996), porté par le même vecteur que celui portant la séquence de l'APR ou par un vecteur différent.

Une surexpression combinée d'APR et de SAT permet d'obtenir une augmentation accrue en glutathion et cystéine.

Les figures et exemples suivants illustrent l'invention sans en limiter la portée.

### LEGENDE DES FIGURES

La figure 1 représente une carte de restriction du vecteur pWP280.
La figure 2 représente une carte de restriction du vecteur pBIOS298.
La figure 3 représente une carte de restriction du plasmide pPS103, contenant une séquence d'APR de *Lemna minor* associée à un promoteur actine du riz.
La figure 4 représente une carte de restriction du vecteur pPS106, contenant une séquence d'APR de *Lemna minor* associée à un promoteur HMWG.
La figure 5 est un graphe montrant la mesure de l'activité de l'APR sur cals de maïs transformés par le construit promoteur actine-intron ∼ APR.
La figure 6 est un graphe montrant la mesure de l'activité de l'APR sur feuilles de plantes Arabidopsis transformées (pro35S∼APR).
La figure 7 est un graphe rapportant les mesures des teneurs en cystéine et glutathion à partir de feuilles de plantes *A. thaliana* surexprimant l'APR (construit pro35S∼APR).
La figure 8A est un graphe rapportant la mesure de l'activité de l'APR sur cals de maïs transformés par le construit promoteur actine-intron-APR.
La figure 8B est un graphe rapportant les mesures des teneurs en cystéine et glutathion à partir de cals de maïs surexprimant l'APR.

### EXEMPLES

### Exemple 1 : Construction d'un vecteur contenant l'ADNc de l'APR de Lemna minor

### 1. Obtention de la séquence d'ADNc de l'APR de Lemna minor

### a) Purification de /'APR

40 g de plantes *Lemna minor* sont homogénéisées en présence de 5'AMP. L'homogénat est centrifugé, et le surnageant traité avec du (NH₄)₂SO₄ (sulfate d'ammonium) jusqu'à une concentration de 30 %. Les protéines précipitées sont recueillies par centrifugation et dissoutes dans un tampon contenant du 5'AMP. La solution est passée sur une colonne de chromatographie contenant du Sephacryl S-300HR. Les fractions présentant une activité enzymatique de 50 % ou plus de la fraction plus active sont soumises à un fractionnement chromatographique sur Mono Q HR, Phenyl-Superose et Superose 12 HR. Les fractions les plus actives éluées de la dernière colonne chromatographique sont concentrées à un volume de 50 µl et appliquées sur un gel de polyacrylamide en condition native. Après électrophorèse, le gel est coupé en segments de 2 mm. Les segments sont élués et les éluats analysés quant à l'activité enzymatique de l'APR. Les éluats contenant les plus grandes activités enzymatiques sont soumis à une séparation par SDS-PAGE. Les gels sont colorés et les protéines sont soumises à un séquençage d'acides aminés.

### b) Isolement de l'ADNc de l'APR :

La région centrale de l'APR a été obtenue à partir de l'ARN de L. *minor* par RT-PCR en utilisant la séquence des domaines conservés entre l'APS réductase de *A. thaliana* et les PAPS réductase de bactéries et de levure, et le motif thioredoxine pour synthétiser des amorces oligonucléotidiques. Le fragment amplifié de 750 paires de base a été complètement séquencé sur ses deux brins. Des amorces spécifiques du gène ont été synthétisées pour une 5'- et 3'-RACE à partir de cette information de séquence. Pour la 5'-RACE, le premier brin d'ADNc a été synthétisé à partir de 0,5 µg d'ARN poly(A) en utilisant une amorce spécifique du gène SP1 (5'-ACTGGTCCTTGCGCTGGCCG-3' (SEQ ID n° 3)), la transcriptase inverse du virus de la leucémie murine de Moloney et un mélange de desoxynucléotides. L'hybride ARNm-ADNc a été ensuite purifié des amorces non incorporées et des nucléotides par le système "GlassMax DNA Isolation Matrix System" (Life Technologies, Inc). Pour l'adaptateur, 10 pmoles d'amorces d'ancrage oligo d(T) (AP, 5'-GACCACGCGTATCGATGTCGACT₁₆-3' (SEQ ID n° 4)) et d'amorces inverses d'ancrage (APrev, 5'-GTCGACATCGATACGCGTGGTC-3' (SEQ IDn° 5)) ont été mis à hybrider. Ensuite, une ligature des extrémités franches de l'adaptateur et de l'hybride ARN-ADNc a été effectuée à 15°C pendant une nuit en utilisant 400 unités de ligase T4 (BioLabs).

L'amplification par PCR suivante a été réalisée avec l'amorce APrev et l'amorce SP2 "nested" (5'-GTGATCCAGGCTCTGAGGCC-3' (SEQ ID n° 6)). Les produits de PCR ont ensuite été séparés sur un gel d'agarose. Pour la seconde amplification par PCR, les amorces APrev et la troisième amorce SP3 "nested" (5'-TCTGAGAGCTCTTCTAAGGGGC-3' (SEQ ID n° 7)) ont été utilisées. La bande majeure (d'approximativement 640 paires de base) sur le gel d'agarose a été utilisée comme matrice. Le produit de PCR a été séparé sur un gel d'agarose et isolé avec le système "GlassMax DNA Isolation Matrix System" (Life Technologies, Inc). L'ADN a été cloné dans un vecteur pCR 2.1 en utilisant le kit de clonage original TA (Invitrogen) suivant les instructions du fabricant.

Pour la 3'-RACE, le premier brin d'ADNc a été synthétisé à partir de 0,5 µg d'ARN poly(A) en utilisant l'amorce d'ancrage oligo d(T), la transcriptase inverse A-MLV, et le mélange de desoxynucleotides. La première amplification par PCR a ensuite été directement mise en oeuvre sans étape de purification supplémentaire, en utilisant l'AP et l'amorce spécifique du gène SP4 (5'-ACCAGGACAGCACGAGAGGG-3' (SEQ ID n° 8)). Le produit de PCR a été séparé par électrophorèse sur gel d'agarose.

Pour la seconde amplification par PCR, la bande sur le gel d'agarose a été utilisée comme matrice avec l'amorce SP5 "nested" (5'-AAGGTGGTGGTGGGAGGACG-3' (SEQ ID n° 9)) et l'AP. Après la séparation des produits de PCR sur un gel d'agarose, la bande majeure (approximativement 560 paires de base) a été purifiée en utilisant le système "GlassMax DNA Isolation Matrix System" (Life Technologies, Inc). L'ADN a été cloné dans un vecteur pCR 2.1 en utilisant le kit de clonage original TA (Invitrogen) suivant les instructions du fabricant.

L'ADNc de l'APR de *Lemna* contient 1377 paires de base (SEQ ID n° 1) et présente un cadre de lecture ouvert (ORF) codant pour une protéine de 459 acides aminés (SEQ ID n° 2). La séquence d'acides aminés déduite contient une région de 30 résidus de Ser-69 à Glu-98 qui est identique à la séquence déterminée par séquençage N-terminal de l'enzyme purifiée (exemple 1 a ci-dessus).

### 2. Préparation des cassettes d'expression

Les techniques de clonage standard pouvant être utilisées pour la réalisation des construits sont décrites dans Sambrook J. et al., Molecular cloning, A laboratory manual, Second edition cold spring harbor laboratory press, 1989.

### a) APR∼35S pour la transformation d'Arabidospsis thaliana

L'APR de Lemna a été cloné par RT-PCR (Sambrook et al., 1989) à partir d'ARN total isolé de plantes entières de Lemna. L'amorce 5' utilisée CGTCTAGAGGCCATGGCGGCCGCAGCAGCAACTATGGCG (SEQ ID n° 10) a généré un site Xbal / Ncol (le site Ncol étant créé au niveau du codon d'initiation ATG). L'amorce 3' AAAGAATTCCACTCTAGAAGCGAAGAGAAGAGGC (SEQ ID n° 11) spécifique de la région 3' non traduite de l'APR de Lemna a généré pour sa part un site EcoRI / Xbal. La séquence d'ADNc de l'APR de Lemna isolée contient une phase ouverte de lecture de 1380 paires de bases comprenant un peptide de transit chloroplastique putatif.

Cet ADNc amplifié a ensuite été cloné dans un vecteur pBluescript SK (Stratagene), aux sites de restriction Xba1. Ce clone, contenant la phase ouverte de lecture de l'APR de Lemna, a été confirmé par séquençage.

La stratégie de clonage du construit promoteur 35S∼APR∼terminateur est basée sur l'utilisation du vecteur pRTL2-GUS/NiaDBamHI (Carrington et al., 1991- The plant cell, 3, 953-962). Ce vecteur contient le promoteur double 35S, la séquence TEV non traduite (Carrington et Freed, 1990. Journal Virol., 64, 1590-1597), le gène GUS, une séquence cible nucléaire (Nia) et le signal polyA 35S. La séquence GUS/Nia de ce vecteur a été remplacée par la séquence APR Lemna par coupure aux sites de restriction Ncol et Xbal et ligature en présence d'ADN ligase T4.

Le construit ainsi généré a été ensuite sous-cloné dans le vecteur binaire pMON505 (Rogers et al., 1987. Methods in enzymology, 153, 253-277) en utilisant le site Hindlll du site multiple de clonage de pMON505 et les sites Hindlll des extrémités 5' et 3' dudit construit. Ce vecteur binaire est utilisé pour transformer la souche *Agrobacterium tumefaciens* GV3101 (Koncz and Schell, 1986. Mol. Gen. Genet., 204, 383-396), destinée à la transformation d'*Arabidopsis thaliana* décrite ultérieurement.

### b) APR promoteur actine (pPS103) pour la transformation du maïs

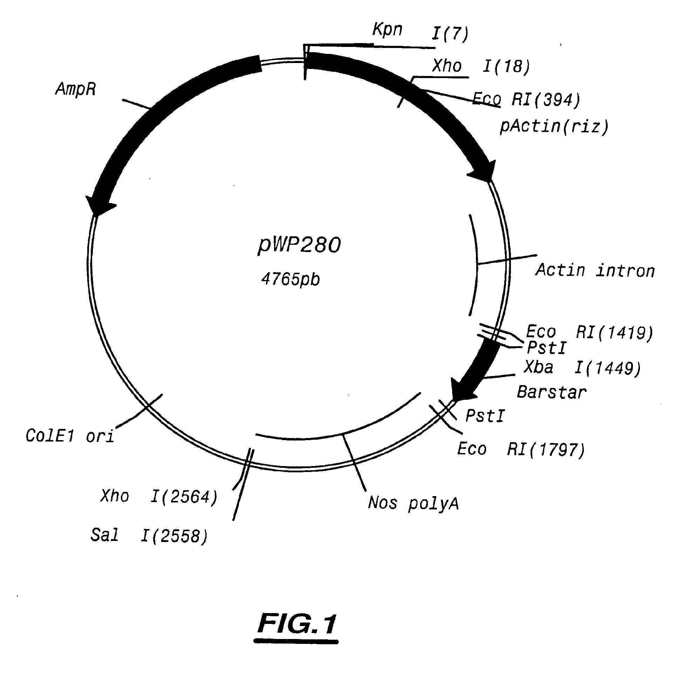

Le clonage du construit promoteur actine∼APR∼Nos 3' utilise comme plasmide de base le plasmide pWP280 contenant la cassette pActin-intron∼Barstar∼Nos polyA (Figure 1), dans laquelle on remplace le fragment Barstar par le fragment APR.

Le plasmide de base a été obtenu selon les étapes suivantes : le gène barstar (Hartley, 1988, J. Mol. Biol., 202, 913-915) a été transféré en tant que fragment Xbal/Hincll dans un site Xbal/Smal du plasmide pW90, dérivé du plasmide pJIT30 décrit par Guérineau et al, 1990, Plant Mol. Biol., 15 :127-136) (promoteur 35ScaMV remplacé par le promoteur double 35S et la région polylinker entre les sites Xbal et EcoRI remplacée par les sites Spel, BamHI, SmaI et PstI). La région polyA CaMV du plasmide obtenu est remplacée par la région polyA de pED23 (Dale et al., 1990, Gene, 91 :79-85) formant le plasmide pWP266. La région promotrice double 35S CaMV est enfin remplacée par le promoteur actine du riz et l'intron issu de pCOR113 (Mc Elroy et al., 1991, Mol. Gen. Genet., 231 :150-160) formant le plasmide pWP280.

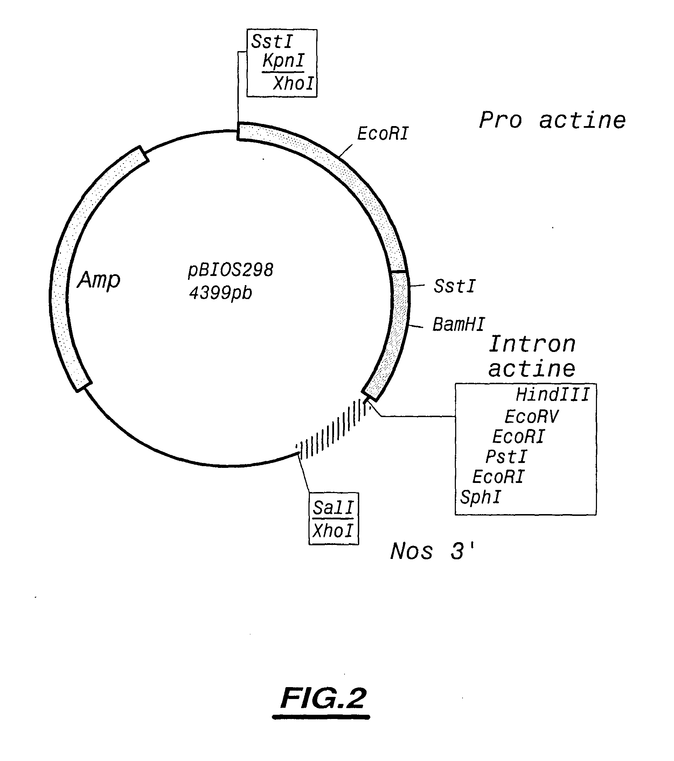

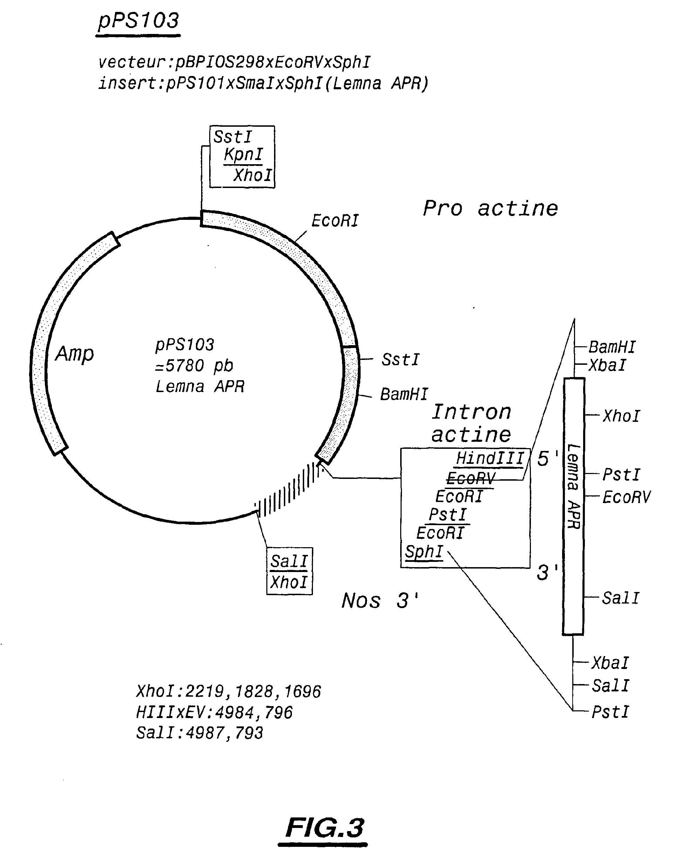

Ce plasmide pWP280 a ensuite été délété de son fragment barstar par digestion avec l'enzyme Pstl, puis religaturé sur lui-même pour donner le vecteur pBIOS298 contenant le fragment promoteur actine-intron-site de clonage multiple-terminateur Nos3' (Figure 2). Celui-ci est ensuite clivé au niveau des sites EcoRV et Sphl du site de clonage multiple, pour y insérer le fragment contenant la séquence ADNc de l'APR de Lemna (1380 pb), obtenu parallèlement par digestion du vecteur pPS101 (dérivé du vecteur pUCAP décrit par van Engelen F.A. et al., 1995, Transgenic Research, 4, 288-290, par insertion de la séquence APR au site multiple de clonage) avec les enzymes Smal et Sphl. Le vecteur et l'insert sont mis en présence d'ADN ligase T4 pour l'étape de ligature au niveau des sites Sphl et EcoRV (les coupures Smal et EcoRV ayant généré des extrémités à bouts francs), conduisant au vecteur pPS103 (Figure 3).

### c) APR promoteur HMWG (pPS106) pour la transformation du maïs

Le vecteur pBL 3214 est dérivé d'un plasmide pBluescript II SK+ (Stratagene) dans lequel on a inséré, par le choix d'enzymes de restriction spécifiques à la portée de l'homme du métier, la séquence promotrice HMWG (High Molecular Weight Glutenin) de blé (Robert et al., 1989, Plant Cell, 1 : 569-578), spécifique de l'albumen des semences et une séquence terminatrice 3' Nos de *Agrobacterium tumefaciens* (Depicker et al., 1982, Mol. Appl. Genet. 1 : 561-573).

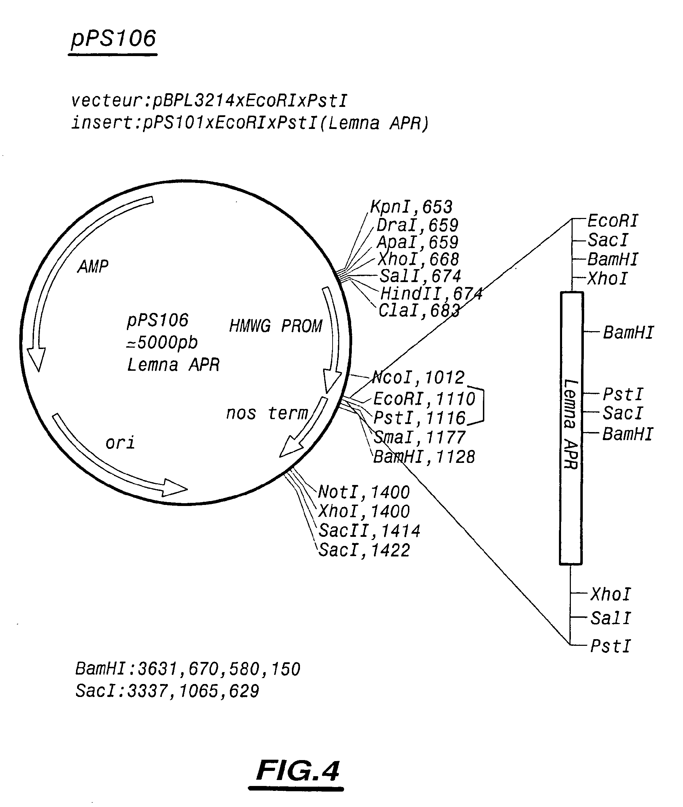

Ce vecteur a été clivé aux sites EcoRI et Pstl du site de clonage multiple, pour y insérer le fragment contenant l'ADNc de l'APR de Lemna, obtenu parallèlement à partir du vecteur pPS101 digéré par les mêmes enzymes EcoRI et PstI. La ligature du vecteur et de l'insert est réalisée en présence d'ADN LigaseT4, pour l'obtention du vecteur pPS106 (Figure 4).

### Exemple 2 : Transformation des plantes

### 1. Transformation du maïs

### a) Canon à particules

La méthode utilisée repose sur l'utilisation d'un canon à particules identique à celui décrit par J. Finer (1992). Les cellules cibles sont des cellules indifférenciées en divisions rapides ayant conservé une aptitude à la régénération de plantes entières. Ce type de cellules compose le cal embryogène (dit de type II) de maïs. Ces cals sont obtenus à partir d'embryons immatures de génotype Hill selon la méthode et sur les milieux décrits par Armstrong (Maize Handbook ; 1994 M. Freeling, V. Walbot Eds ; pp.665-671). Ces fragments des cals d'une surface de 10 à 20 mm² ont été disposés, 4h avant bombardement, à raison de 16 fragments par boite au centre d'une boîte de Petri contenant un milieu de culture identique au milieu d'initiation, additionné de 0,2 M de mannitol + 0,2 M de sorbitol. Les plasmides décrits dans les exemples précédents et portant les séquences APR à introduire, sont purifiés sur colonne Qiagen^{R} en suivant les instructions du fabricant. Ils sont ensuite précipités sur des particules de tungstène (M10) en suivant le protocole décrit par Klein (1987). Les particules ainsi enrobées sont projetées vers les cellules cibles à l'aide du canon et selon le protocole décrits par J. Finer (1992). Les boîtes de cals ainsi bombardées sont ensuite scellées à l'aide de Scellofrais^{R} puis cultivées à l'obscurité à 27°C. Le premier repiquage a lieu 24h après, puis tous les quinze jours pendant 3 mois sur milieu identique au milieu d'initiation additionné d'un agent sélectif. On obtient après 3 mois ou parfois plus tôt, des cals dont la croissance n'est pas inhibée par l'agent sélectif, habituellement et majoritairement composés de cellules résultant de la division d'une cellule ayant intégré dans son patrimoine génétique une ou plusieurs copies du gène de sélection. La fréquence d'obtention de tels cals est d'environ 0,8 cal par boîte bombardée.

Ces cals sont identifiés, individualisés, amplifiés puis cultivés de façon à régénérer des plantules, en modifiant l'équilibre hormonal et osmotique des cellules selon la méthode décrite par Vain et al. (1989). Ces plantes sont ensuite acclimatées en serre où elles peuvent être croisées pour l'obtention d'hybrides ou autofécondées.

### b) Transformation par Agrobacterium

Une autre technique de transformation utilisable dans le cadre de l'invention utilise *Agrobacterium tumefaciens*, selon le protocole décrit par Ishida et al (1996), notamment à partir d'embryons immatures prélevés 10 jours après la fécondation. Tous les milieux utilisés sont référencés dans la référence citée. La transformation débute avec une phase de co-culture où les embryons immatures des plantes de maïs sont mis en contact pendant au moins 5 minutes avec *Agrobacterium tumefaciens* LBA 4404 contenant les vecteurs superbinaires. Le plasmide superbinaire est le résultat d'une recombinaison homologue entre un vecteur intermédiaire porteur de l'ADN-T contenant le gène d'intérêt et/ou le marqueur de sélection dérivé des plasmides décrits dans les exemples précédents, et le vecteur pSB1 de Japan Tobacco (EP 672 752) qui contient : les gènes virB et virG du plasmide pTiBo542 présent dans la souche supervirulente A281 *d'Agrobacterium tumefaciens* (ATCC 37349) et une région homologue retrouvée dans le vecteur intermédiaire permettant cette recombinaison homologue. Les embryons sont ensuite placés sur milieu LSAs pendant 3 jours à l'obscurité et à 25°C. Une première sélection es: effectuée sur les cals transformés : les cals embryogènes sont transférés sur milieu LSD5 contenant de la phosphinotricine à 5 mg/l et de la céfotaxime à 250 mg/l (élimination ou limitation de la contamination par *Agrobacterium tumefaciens*). Cette étape est menée 2 semaines à l'obscurité et à 25°C. La deuxième étape de sélection est réalisée par transfert des embryons qui se sont développés sur milieu LSD5, sur milieu LSD10 (phosphinotricine à 10 mg/l) en présence de céfotaxime, pendant 3 semaines dans les mêmes conditions que précédemment. La troisième étape de sélection consiste à exciser les cals de type I (fragments de 1 à 2 mm) et à les transférer 3 semaines à l'obscurité et à 25°C sur milieu LSD 10 en présence de céfotaxime.

La régénération des plantules est effectuée en excisant les cals de type I qui ont proliféré et en les transférant sur milieu LSZ en présence de phosphinotricine à 5 mg/l et de céfotaxime pendant 2 semaines à 22°C et sous lumière continue.

Les plantules ayant régénéré sont transférées sur milieu RM + G2 contenant 100mg/l d'Augmentin pendant 2 semaines à 22°C et sous illumination continue pour l'étape de développement. Les plantes obtenues sont alors transférées au phytotron en vue de leur acclimatation.

### 2. Transformation d'Arabidopsis

*Arabidopsis thaliana* peut être facilement transformée avec un vecteur décrit à l'exemple 1, selon la méthode décrite dans Bechtold et al, 1993, basée sur l'infiltration sous vide de plantes d'*Arabidopsis* par une souche d'*Agrobacterium* contenant un vecteur binaire.

Selon cette méthode, des souches d'*Agrobacterium* portant un système de vecteurs binaires ont été cultivées jusqu'à une densité optique de 1,0 à 600 nm, puis resuspendues dans une solution contenant 5 % de saccharose, 10 mM de MgCl₂ et 0,005 % de Silwet L-77 (# VIS-01 ; LEHLE SEEDS, Round Rock, Texas, USA). Des plantes *A. thaliana* ont été infiltrées sous vide avec une suspension d'*Agrobacterium*, pendant 5 minutes. Les semences T1 obtenues à partir de ces plantes infiltrées sous vide ont été vernalisées, stérilisées et étalées sur un milieu MS. Les plantes obtenues ont ensuite été transférées en terre et cultivées dans des conditions standards (20°C, 16 illuminations par jour) dans un incubateur. Les semences T2 obtenues à partir de ces plantes ont été recueillies pour analyse (Kost B. et al, The Plant Journal, 1998, n° 16, pp 393-401).

### 3. Surexpression de l'APR dans les plantes transformées :

L'activité APR a été mesurée dans les plantes transformées ci-dessus, en comparaison avec des plantes contrôles, selon la méthode décrite dans Kopriva et al, 1999.

Le matériel végétal (cals de maïs et feuilles d'Arabidopsis) a été extrait dans un tampon de 50 mM de NaKPO₄ (pH 8), additionné de 30 mM Na₂SO₃, 0,5 mM 5'-AMP, et 10 mM DTE (Imhof, 1994), en utilisant un homogénéisateur en verre. L'activité APR a été mesurée dans les extraits, au moyen de la production de [³⁵S] sulfite, évaluée comme la radioactivité volatile acide formée en présence de [³⁵S] APS et DTE (Brunold et Suter, 1990). Les concentrations protéiques dans les extraits ont été déterminées selon la méthode de Bradford (1976), avec la sérum albumine de boeuf comme standard.

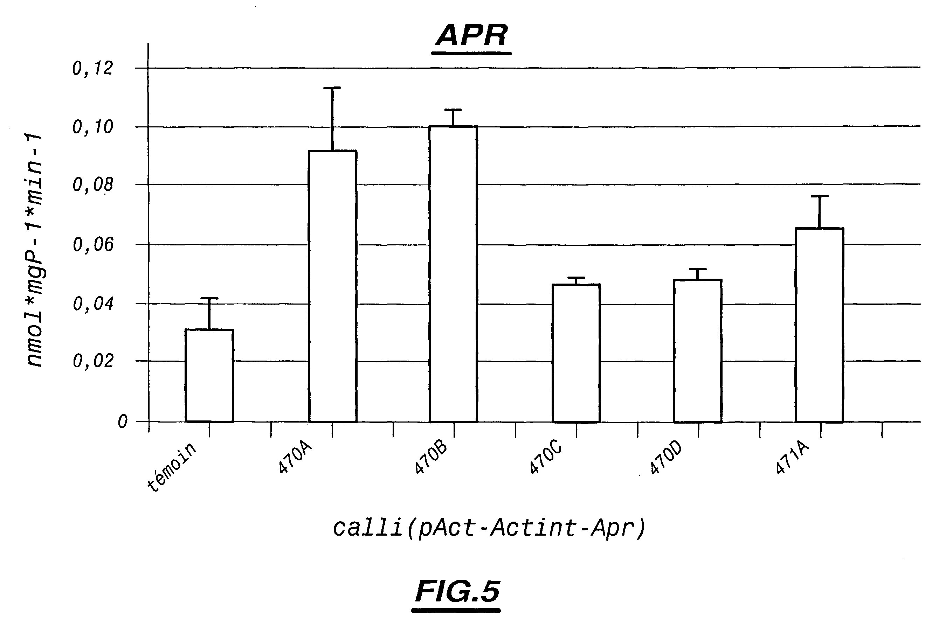

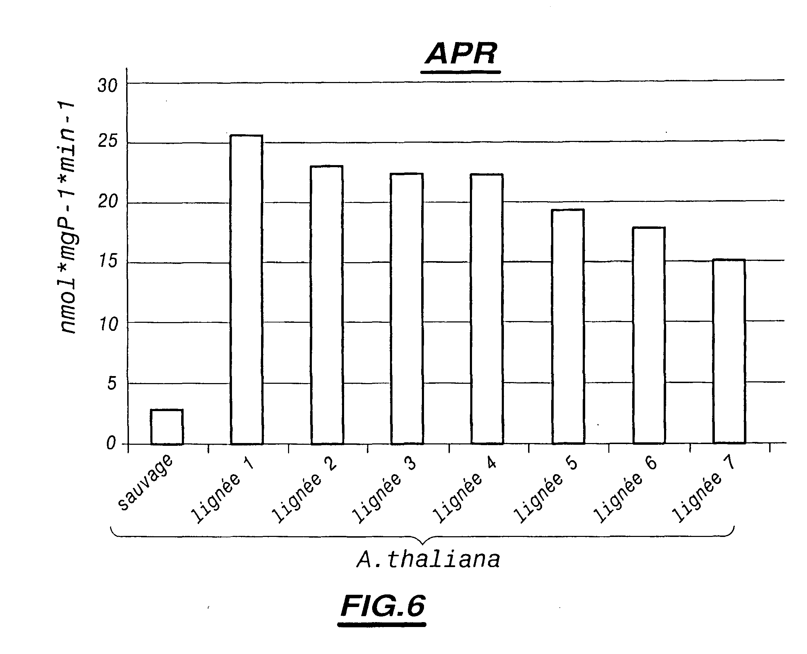

Comme le montrent les figures 5 et 6, les plantes transformées (maïs et Arabidopsis respectivement) présentent une surexpression en enzyme APR.

### Exemple 3 : Obtention de plantes enrichies en cystéine et glutathion.

### Méthode :

Les teneurs en cystéine et glutathion de différents organes de l'*Arabidopsis* transformée à l'exemple 2.2 et du maïs transformé à l'exemple 2.1, ont été mesurées par la méthode de Ruegsegger et al, 1992, après réaction avec du monobromobimane. Cette méthode peut être résumée comme suit :

Les thiols sont séparés et quantifiés par HPLC en phase inverse après réduction avec NaBH₄ et réaction avec du monobromobimane qui se lie aux thiols et envoie un signal fluorescent. Les extraits sont centrifugés pendant 30 minutes à 30 000 g et à 4°C. 600 µl de CHES 0,2 M (pH 9,3) et 100 µl de solution NaBH₄ 40 mM fraîchement préparés ont été ajoutés à 400 µl de surnageant. Le contrôle négatif était un milieu d'extraction. Le mélange a été maintenu dans la glace pendant 30 minutes. Pour la réaction, 300 µl de ce mélange ont été ajoutés à 15 µl de monobromobimane 15 mM dissous dans de l'acétonitrile et maintenu dans le noir à température ambiante pendant 15 minutes. La réaction a été stoppée par l'addition de 250 µl d'acide acétique à 5 % (volume/volume). Les échantillons ont été centrifugés pendant 10 minutes à 16 000 g et 4°C et le surnageant non dilué a été utilisé pour la mesure de cystéine. Une dilution de 20 fois avec de l'acide acétique à 2,5 % (volume/volume) a été utilisée pour la mesure de glutathion GSH. Les échantillons ont ensuite été analysés selon la méthode de Schupp et Rennenberg, 1988 sur un système HPLC Gold (Beckman) avec une colonne Nucleosil 100-S C₁₈ (4,0 x 250 mm ; Machereu-Nagel Oepsingen Switzerland) et un détecteur de fluorescence SFM 25 (Kontron, Zurich, Switzerland).

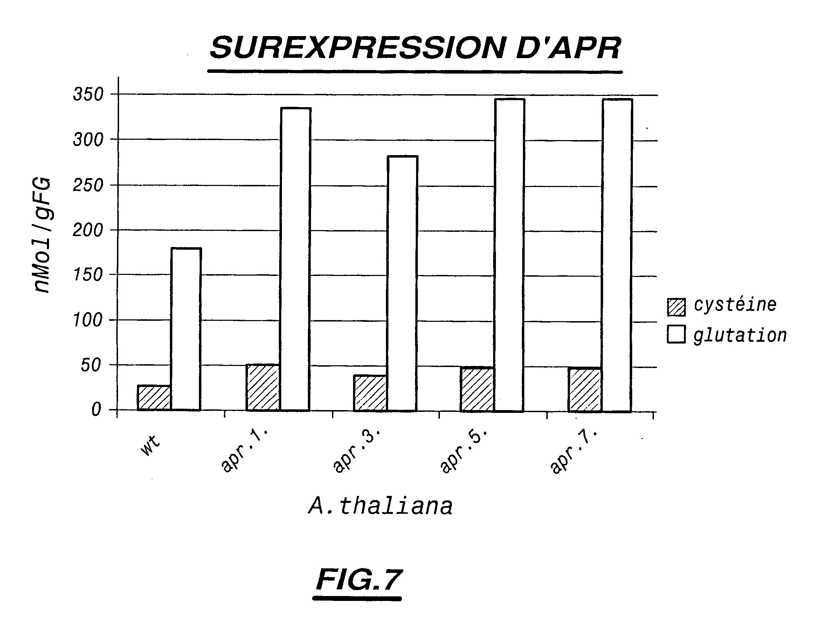

Les résultats des mesures des teneurs en cystéine et glutathion à partir de feuilles d'*A. thaliana* surexprimant l'APR sont exprimés sur la figure 7.

On observe une augmentation significative de ces teneurs en cystéine et glutathion par rapport à la plante sauvage non transformée.

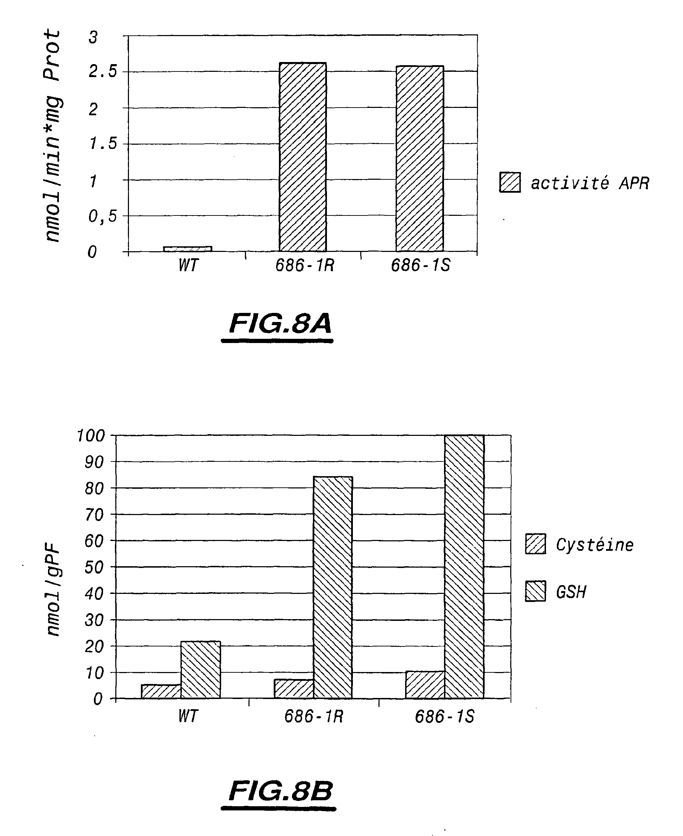

Les teneurs en cystéine et glutathion de cals de maïs transformés et non transformés ont également été mesurées selon le même protocole. Les résultats sont exprimés sur les figures 8A et 8B. On observe, de façon corrrélée à la surexpression de l'APR, une augmentation significative de ces teneurs en cystéine et glutathion par rapport aux cals non transformés.

Des mesures ont également été effectuées sur les plantules obtenues à partir de ces cals transformés et ont donné des résultats positifs.

### REFERENCES

- An et al, 1986, Plant Physiol., 81:86-91
- Armstrong (1994). Maize Handbook ; Freeling M., Walbot V. Eds ; 665-671.
- Bechtold et al, 1993, C.R. Acad. Sci. Paris, Life sciences, 316:1194-1199
- Bick et Leustek, 1998, Current opinion in Plant Biology, 1:240-244
- Bradford 1976, Anal. Biochem, 72:248-254
- Brunold et al, 1997, Prog. Bot., 58:164-186
- Brunold et Suter, 1990, Adenosine 5'-posphosulfate sulfotransferase, I, P Lea, ed. Methods in Plant biochemistry vol. 3, Academic Press, London 339-343
- Carrington et al., 1991, The plant cell, 3, 953-962
- Carrington et Freed, 1990, Journal Virol., 64, 1590-1597
- Chupeau et Coll., 1989, Biotechnology, 7(5):503-508
- Dale et al., 1990, Gene, 91 :79-85
- Depicker et al., 1982, Mol.Appl. Genet. 1 : 561-573).
- Finer J., (1992). Plant Cell Report, 11 : 323-328.
- Fromm M. et al., 1990, Biotechnology, 8:833-839
- Guerche et al, (1987). Mol. Gen. Genet. 206:382
- Guerineau et al, (1990), Plant. Mol. Biol. 15:127-136
- Gutierrez-Marcos et al, 1996, PNAS, vol. 93, pp 13377-13382
- Hartley et al., (1988), J. Mol. Biol., 202, 913-915
- Heiss et al, 1999, Plant Mol. Biol., 39:847-857
- Herrera-Estrella et al, 1983,EMBO J. 2, 987-995
- Imhof, 1994, Diploma work, Institute for Plant Physiology of the University of Berne
- Ishida et al., (1996), Nature Biotechnology, 14 :745-750
- Kay et al, 1987, Science, 236:1299-1302
- Klein, (1987). Nature, 327 : 70-73.
- Koncz and Schell, 1986. Mol. Gen. Genet., 204, 383-396
- Kopriva et al, 1999, The Plant Journal, 20(1), 37-44
- Kost B. et al, The Plant Journal, 1998, n° 16, pp 393-401
- May et al, 1998, Journal of Exp. Bot., 49:649-667
- Mc Elroy et al., 1991, Mol. Gen. Genet., 231 :150-160
- Murillo et al, 1995, Arch Biochem Biophys 323:195-204
- Neuhaus et Coll., 1987, Theoretical and Applied Genetics, 75(1):30-36
- Rennenberg et al, 1990, SPB Academic Publishing, The Hague, The Netherlands pp 53-65
- Rennenberg et al, 1994, Prog Bot, 55:144-156
- Robert et al., 1989, Plant Cell, 1 : 569-578,
- Rogers et al., 1987. Methods in enzymology, 153, 253-277
- Rüegsegger et al, 1992, Plant Physiol. n° 99, pp 428-433
- Saito et al, 1995, J. Biol. Chem. 270:16321-6
- Sambrook J. et al., Molecular cloning, A laboratory manual, Second edition cold spring harbor laboratory press, 1989
- Schmidt et al, 1992, Annu. Rev. Plant Physiol. Plant Mol. Biol., 43:325-349
- Schocher et Coll., 1986, Biotechnology 4:1093-1096
- Schupp et Rennenberg, 1988, Plant Sci 57:113-117
- Vain et al., 1989, Plant Cell Tissue and organ culture, 18:143-151
- van Engelen F.A. et al., 1995, Transgenic Research, 4, 288-290,
- Watson et al, 1994, ADN recombinant, Ed. De Boeck université, 273-292

## Revendications

1. Procédé d'obtention d'une plante présentant une teneur enrichie en cystéine et/ou glutathion, comprenant les étapes consistant à :
- transformer au moins une cellule de plante avec un vecteur contenant une cassette d'expression comprenant un acide nucléique de séquence SEQ ID N°1 codant pour une Adénosine 5'-Phosphosulfate Réductase (APR) de *Lemna minor* ;
- cultiver la cellule ainsi transformée de manière à générer une plante contenant dans son génome ladite cassette d'expression.

2. Procédé selon la revendication 1, dans lequel la cellule de plante est aussi transformée avec un acide nucléique codant pour une Sérine Acetyltransférase (SAT).

3. Procédé selon l'une des revendications 1 à 2, dans lequel la cassette d'expression comprend un promoteur permettant l'expression de l'acide nucléique codant pour l'APR de *Lemna minor* dans les semences de la plante obtenue.

4. Procédé selon la revendication 3, dans lequel ledit promoteur est le promoteur HMWG de blé.

5. Plante ou partie de plante, susceptible d'être obtenue par le procédé selon l'une quelconque des revendications 1 à 4, et contenant une cassette d'expression comprenant un acide nucléique de séquence SEQ ID N°1 codant pour l'enzyme APR de *Lemna minor.*

6. Plante selon la revendication 5 contenant une cassette d'expression comprenant un acide nucléique de séquence SEQ ID N°1 codant pour l'enzyme APR de *Lemna minor,* **caractérisée en ce qu'**il s'agit du maïs.

7. Partie de plante selon la revendication 5 contenant une cassette d'expression comprenant un acide nucléique de séquence SEQ ID N°1 codant pour l'enzyme APR de *Lemna minor*, **caractérisée en ce qu'**il s'agit du grain.

8. Plante transgénique hybride, contenant une cassette d'expression comprenant un acide nucléique de séquence SEQ ID N°1 codant pour l'enzyme APR de *Lemna minor,* susceptible d'être obtenue par le croisement d'au moins une plante telle que définie dans l'une quelconque des revendications 5 ou 6.

9. Utilisation d'un acide nucléique de séquence SEQ ID N°1 codant pour une Adénosine 5'-Phosphosulfate Réductase (APR) de *Lemna minor,* pour l'obtention d'une plante transgénique présentant une teneur enrichie en cystéine et/ou gluthation, par rapport à une plante non transformée.

10. Utilisation d'une plante ou partie de plante telle que définie dans l'une des revendications 5 à 8, contenant une cassette d'expression comprenant la séquence nucléotidique SEQ ID N°1 codant pour l'enzyme APR de *Lemna minor,* pour la préparation de produits dérivés, notamment de produits pour l'alimentation animale.

11. Produits, tels que produits alimentaires, contenant une plante ou partie de plante telle que définie dans l'une des revendications 5 à 8 contenant une cassette d'expression comprenant un acide nucléique de séquence SEQ ID N°1 codant pour l'enzyme APR de *Lemna minor.*

12. Acide nucléique codant pour l'enzyme APR de *Lemna minor,* ledit acide nucléique comprenant la séquence nucléotidique SEQ ID n° 1.

13. Cassette d'expression comprenant la séquence nucléotidique SEQ ID n° 1, codant pour l'enzyme APR de *Lemna minor* liée de manière opérante à des éléments permettant son expression et éventuellement sa régulation.

14. Vecteur d'expression dans lequel est insérée une cassette d'expression selon la revendication 13.

15. Cellule hôte transformée avec un vecteur selon la revendication 14.

16. Maïs contenant un acide nucléique selon la revendication 12.

## Claims

1. Method for obtaining a plant exhibiting an enriched cysteine and/or glutathione content, comprising the steps consisting in:
- transforming at least one plant cell with a vector containing an expression cassette comprising a nucleic acid having the sequence SEQ ID No. 1 encoding an adenosine 5'-phosphosulphate reductase (APR) of *Lemna minor*;
- culturing the cell thus transformed so as to generate a plant containing said expression cassette in its genome.

2. Method according to Claim 1, in which the plant cell is also transformed with a nucleic acid encoding a serine acetyltransferase (SAT).

3. Method according to either of Claims 1 and 2, in which the expression cassette comprises a promoter which allows expression of the nucleic acid encoding the APR of *Lemna minor* in the seeds of the plant obtained.

4. Method according to Claim 3, in which said promoter is the wheat HMWG promoter.

5. Plant or part of a plant, which can be obtained using the method according to any one of Claims 1 to 4, and which contains an expression cassette comprising a nucleic acid having the sequence SEQ ID No. 1 encoding the APR enzyme of *Lemna minor.*

6. Plant according to Claim 5, which contains an expression cassette comprising a nucleic acid having the sequence SEQ ID No. 1 encoding the APR enzyme of *Lemna minor,* **characterized in that** it is maize.

7. Part of a plant according to Claim 5, which contains an expression cassette comprising a nucleic acid having the sequence SEQ ID No. 1 encoding the APR enzyme of *Lemna minor,* **characterized in that** it is the grain.

8. Hybrid transgenic plant, which contains an expression cassette comprising a nucleic acid having the sequence SEQ ID No. 1 encoding the APR enzyme of *Lemna minor* and which can be obtained by crossing at least one plant as defined in either one of Claims 5 and 6.

9. Use of a nucleic acid having the sequence SEQ ID No. 1 encoding an adenosine 5'-phosphosulphate reductase (APR) of *Lemna minor,* to obtain a transgenic plant exhibiting an enriched cysteine and/or glutathione content compared with a nontransformed plant.

10. Use of a plant or part of a plant as defined in one of Claims 5 to 8, which contains an expression cassette comprising the nucleotide sequence SEQ ID No. 1 encoding the APR enzyme of *Lemna minor,* for preparing derived products, in particular products for animal feed.

11. Products, such as food products, containing a plant or part of a plant as defined in one of claims 5 to 8 which contains an expression cassette comprising a nucleic acid having the sequence SEQ ID No. 1 encoding the APR enzyme of *Lemna minor.*

12. Nucleic acid encoding the APR enzyme of *Lemna minor,* said nucleic acid comprising the nucleotide sequence SEQ ID No. 1.

13. Expression cassette comprising the nucleotide sequence SEQ ID No. 1, encoding the APR enzyme of *Lemna minor,* functionally linked to elements which allow the expression thereof and, optionally, the regulation thereof.

14. Expression vector into which an expression cassette according to Claim 13 is inserted.

15. Host cell transformed with a vector according to Claim 14.

16. Maize containing a nucleic acid according to Claim 12.

## Patentansprüche

1. Verfahren zur Herstellung einer Pflanze mit einem erhöhten Cystein- und/oder Glutathiongehalt, umfassend die folgenden Schritte bestehend aus:
- Transformieren von mindestens einer Pflanzenzelle mit einem Vektor, der eine Expressionskassette umfassend eine Nukleinsäure mit der Sequenz SEQ ID Nr. 1, die für eine Adenosin-5'-phosphosulfat-Reduktase (APR) aus *Lemna minor* codiert, enthält;
- Kultivieren der so transformierten Zelle, um zu einer Pflanze zu gelangen, die diese Expressionskassette in ihrem Genom enthält.

2. Verfahren nach Anspruch 1, wobei die Pflanzenzelle auch mit einer Nukleinsäure, die für eine Serinacetyltransferase (SAT) codiert, transformiert ist.

3. Verfahren nach einem der Ansprüche 1 bis 2, wobei die Expressionskassette einen Promoter umfaßt, der die Expression der für die APR aus *Lemna minor* codierenden Nukleinsäure in den Samen der so erhaltenen Pflanze gestattet.

4. Verfahren nach Anspruch 3, wobei es sich bei dem Promoter um den HMWG-Promoter aus dem Weizen handelt.

5. Pflanze oder Pflanzenteil, die bzw. der nach dem Verfahren gemäß einem der Ansprüche 1 bis 4 erhältlich ist, und die/der eine Expressionskassette umfassend eine Nukleinsäure mit der Sequenz SEQ ID Nr. 1, die für das Enzym APR aus *Lemna minor* codiert, enthält.

6. Pflanze nach Anspruch 5, die eine Expressionskassette umfassend eine Nukleinsäure mit der Sequenz SEQ ID Nr. 1, die für das Enzym APR aus *Lemna minor* codiert, enthält, **dadurch gekennzeichnet, daß** es sich um den Mais handelt.

7. Pflanzenteil nach Anspruch 5, die eine Expressionskassette umfassend eine Nukleinsäure mit der Sequenz SEQ ID Nr. 1, die für das Enzym APR aus *Lemna minor* codiert, enthält, **dadurch gekennzeichnet, daß** es sich um Getreide handelt.

8. Transgene Hybridpflanze, die eine Expressionskassette umfassend eine Nukleinsäure mit der Sequenz SEQ ID Nr. 1, die für das Enzym APR aus *Lemna minor* codiert, enthält, die durch Kreuzen von mindestens einer Pflanze nach einem der Ansprüche 5 oder 6 erhältlich ist.

9. Verwendung einer Nukleinsäure mit der Sequenz SEQ ID Nr. 1, die für eine Adenosin-5'-phosphosulfat-Reduktase (APR) aus *Lemna minor* codiert, für die Erzeugung einer transgenen Pflanze mit einem im Vergleich zu einer nichttransformierten Pflanze erhöhten Cystein- und/oder Glutathiongehalt.

10. Verwendung einer Pflanze oder eines Pflanzenteils nach einem der Ansprüche 5 bis 8, die/der eine Expressionskassette umfassend die Nukleotidsequenz SEQ ID Nr. 1, die für das Enzym APR aus *Lemna minor* codiert, enthält, für die Herstellung von Verarbeitungsprodukten, insbesondere von Produkten für die Tierernährung.

11. Produkte wie Nahrungsmittelprodukte, die eine Pflanze oder einen Pflanzenteil nach einem der Ansprüche 5 bis 8 enthalten, die eine Expressionskassette umfassend eine Nukleinsäure mit der Sequenz SEQ ID Nr. 1, die für das Enzym APR aus *Lemna minor* codiert, enthalten.

12. Nukleinsäure, die für das Enzym APR aus *Lemna minor* codiert, wobei die Nukleinsäure die Nukleotidsequenz SEQ ID Nr. 1 umfaßt.

13. Expressionskassette, die die Nukleotidsequenz SEQ ID Nr. 1, die für das Enzym APR aus *Lemna minor* codiert, in operativer Verknüpfung mit Elementen, die ihre Expression und gegebenenfalls ihre Regulation gestatten, umfaßt.

14. Expressionsvektor, in dem eine Expressionskassette nach Anspruch 13 insertiert ist.

15. Wirtszelle, die mit einem Vektor nach Anspruch 14 transformiert ist.

16. Mais, der eine Nukleinsäure nach Anspruch 12 enthält.
